# EUROPEAN PATENT APPLICATION

(11) **EP 4 156 680 A1**
(43) Date of publication of application: **29.03.2023**
(21) Application number: 21198797.9
(22) Date of filing: 24.09.2021
(51) Int. Cl.: H04N 13/204

(54) **THERMALLY STABLE CAMERA MOUNT**

(71) Applicant: Koninklijke Philips N.V., 5656 AG Eindhoven (NL)
(72) Inventor: DE BOER, Jacob, Eindhoven (NL); KEMP, Marco, Eindhoven (NL)
(74) Representative: Philips Intellectual Property & Standards

(57) **Abstract**

The present invention relates to a mount for a camera arrangement of a medical navigation setup. In order to provide an improved mount requiring less calibration or correction, a mount (10) comprises a body structure (12) provided with a central point (14), which is configured for mounting the body structure to a support, at least two camera suspensions (16) slidably mounted to the body structure at holding portions (18), which holding portions are displaced in relation to the central point, and a fixation device (20) for each of the at least two camera suspensions. Each of the fixation devices comprises a longitudinal holding element (22), which is attached to the respective camera suspensions at a first end (24) and which is attached to the body structure with a fixation (28) at a second end (26). The longitudinal holding element extends from the first end to the second end at least from the holding portion towards the central point. The longitudinal holding element is arranged for providing length compensation for a change of dimension of the body structure due to thermal expansion.

## Description

### FIELD OF THE INVENTION

The present invention relates to mounting cameras, and relates in particular to a mount for a camera arrangement of a medical navigation setup, to a camera arrangement, to a medical imaging system and to a method for holding cameras.

### BACKGROUND OF THE INVENTION

For medical interventions, navigational information can be provided by medical imaging. A further navigating support can be provided by detecting relative positions of tools and subject, for example by registering visual markers on patients and surgical tools. For determining a spatial arrangement, optical cameras can be used, e.g. two or more cameras arranged in a so-called Smart Vision Box (SVB). The stable positions of the cameras are very important for a precise registration. However, it has been shown that calibration for compensating geometric changes due to thermal changes can be cumbersome.

### SUMMARY OF THE INVENTION

There may thus be a need for an improved mount requiring less calibration or correction efforts.

The object of the present invention is solved by the subject-matter of the independent claims; further embodiments are incorporated in the dependent claims. It should be noted that the following described aspects of the invention apply also for the a mount for a camera arrangement of a medical navigation setup, for the camera arrangement, for the medical imaging system and for the method for holding cameras.

According to the present invention, a mount for a camera arrangement of a medical navigation setup is provided. The mount comprises a body structure provided with a central point, which is configured for mounting the body structure to a support. The mount also comprises at least two camera suspensions slidably mounted to the body structure at holding portions, which holding portions are displaced in relation to the central point. The mount further comprises a fixation device for each of the at least two camera suspensions. Each of the fixation devices comprises a longitudinal holding element, which is attached to the respective camera suspensions at a first end and which is attached to the body structure with a fixation at a second end. The longitudinal holding element extends from the first end to the second end at least from the holding portion towards the central point. The longitudinal holding element is arranged for providing length compensation for a change of dimension of the body structure due to thermal expansion.

As an effect, a thermally stable camera mount is provided. The thermal compensated design can be descripted as a passive solution which means that no active elements are required to keep each camera in a stable position. This makes this solution very reliable and cost effective; no additional compensation technologies are needed, and this will also result in a compact design where only the registration via the cameras is needed.

This allows that during a procedure the surgeon or operator can make direct use of the camera setup or camera system, also known as smart vision box, SVB, without stabilization time needed. The possible accuracy is very high to execute precise procedures, also combined with other medical devices or or assisting robotics.

According to an example, at least one of the longitudinal holding elements extends across the central point. The fixation of the longitudinal holding element is attached to the body structure beyond the central point in relation to the respective camera suspensions.

According to an example, with increasing temperature, the longitudinal holding element expands at its first end in a first direction of its extension in relation to the second end. With the fixation, the second end is fixedly mounted to a part of the body structure that expands in a second direction away from the central point. The first and the second direction are opposite to each other such that an expanding of the body structure in the second direction at least partly compensates an expanding of the longitudinal holding element in the first direction.

According to an example, the longitudinal holding element is arranged such that the fixation is arranged with a first distance to the central point and the camera suspension is arranged with a second distance to the central point. A ratio of the first distance and the second distance is based on a ratio of a thermal expansion of the body structure and a thermal expansion of the longitudinal holding element.

According to an example, the longitudinal holding element provides a more stable relative position of the camera suspension in relation to the central point than the holding portions in relation to the body structure central point.

According to an example, the longitudinal holding element is made from a material having a smaller thermal expansion than the body structure.

In an option, the longitudinal holding element is made from a nickel-iron alloy and the body structure is made from Aluminum.

According to an example, two camera suspensions are provided arranged on two ends of a beam-like structure provided by the body structure, which two ends are having the central point between the two ends.

According to an example, the body structure comprises two beams that are provided in a crossing manner providing four beam ends having the central point between the two ends. Four camera suspensions are provided, one arranged on each of the four ends.

According to the present invention, also a camera arrangement is provided. The camera arrangement comprises a mount according to one of the preceding examples. The camera arrangement also comprises a plurality of cameras. Each of the camera suspensions is holding one of the plurality of cameras.

According to the present invention, also a medical imaging system is provided. The medical imaging system comprises a medical imaging arrangement, configured to acquire non-visible image data from a region of interest of a subject. The medical imaging system also comprises a camera arrangement according to one of the preceding examples, configured to acquire visible image data from the region of interest of the subject. Further, the medical imaging system comprises a set of markers that are visible by the medical imaging arrangement and by the camera arrangement. The imaging system is configured to provide a registration of visible images with image data of the medical imaging device based on the markers.

According to an example, the set of markers comprises at least one of the group of skin markers for subject registration, device markers for tracking operating parameters of surgical devices like needle or drilling position tracking and robot markers for tracking and calibrating of robotic operation for accurate positioning and automated movements.

According to the present invention, also a method for holding cameras is provided. The method comprises the following steps: In a first step, a mount for a camera arrangement of a medical navigation setup is provided. The mount comprises a body structure provided with a central point, which is configured for mounting the body structure to a support. The mount also comprises at least two camera suspensions slidably mounted to the body structure at holding portions, which holding portions are displaced in relation to the central point, i.e. with a distance to the central point. The mount further comprises a fixation device for each of the at least two camera suspensions. Each of the fixation devices comprises a longitudinal holding element, which is attached to the respective camera suspension at a first end and which is attached to the body structure with a fixation at a second end. The longitudinal holding element extends from the first end to the second end at least from the holding portion towards the central point. In a second step, the body structure is thermally expanding in a first direction due to increasing thermal loads. In a third step, length compensation is provided by the longitudinal holding element for a change of dimension of the body structure due to thermal expansion.

According to an aspect, thermal compensation is provided in opposite directions with selected materials with different specific thermal expansion numbers. In an example, Invar (trademark of Imphy Alloys) bars/rods and an Aluminum housing (cross holder) is provided.

According to an aspect, the solution is based on thermal expansion in opposite directions which can be fine-tuned to have a very accurate camera position in a large temperature range. This will also avoid stabilization time needed. Direct system use is possible which is very useful where direct need is required.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a mount for a camera arrangement of a medical navigation setup.
Fig. 2 schematically shows a further example of a mount for a camera arrangement of a medical navigation setup.
Fig. 3 shows steps of an example of a method for holding cameras.
Fig. 4 shows an example of a medical imaging system.
Fig. 5 shows a perspective view of a part of a camera arrangement.
Fig. 6 shows a bottom view of the camera arrangement of Fig. 5.
Fig. 7 shows a further perspective view from below of the camera arrangement.
Fig. 8 shows a perspective view from above of the camera arrangement.
Fig. 9 shows a perspective view from above of the camera arrangement mounted to a movable support arm.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

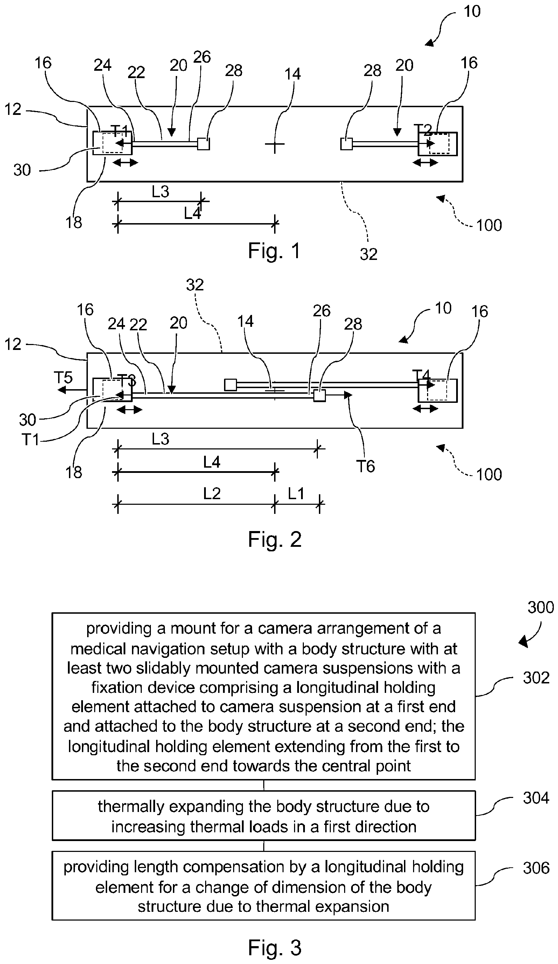

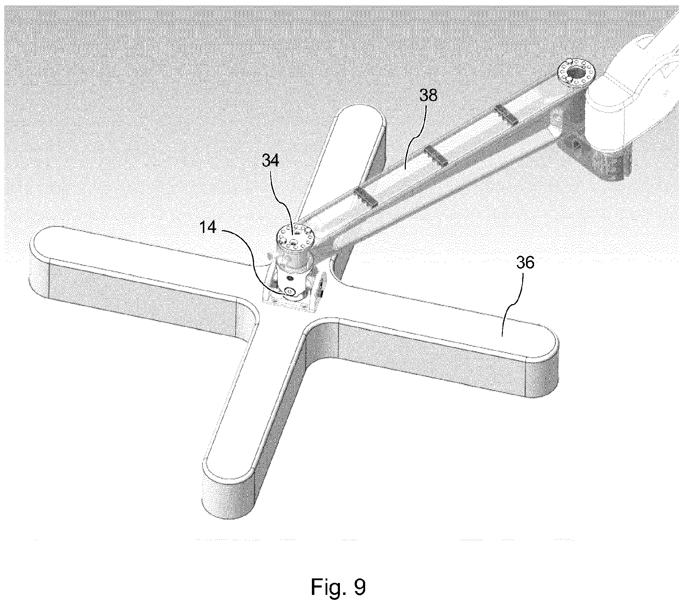

Fig. 1 schematically shows an example of a mount 10 for a camera arrangement of a medical navigation setup. The mount 10 comprises a body structure 12 provided with a central point 14 that is configured for mounting the body structure 12 to a support (also see Fig. 9). The mount 10 also comprises at least two camera suspensions 16 that are slidably mounted to the body structure 12 at holding portions 18. The possibility of sliding is indicated with a double arrow. The holding portions 18 are displaced in relation to the central point 14, for example with distance *L4*. The mount 10 further comprises a fixation device 20 for each of the at least two camera suspensions 16. Each of the fixation devices 20 comprises a longitudinal holding element 22 having a first end 24 and a second end 26. The longitudinal holding element 22 is attached to the respective camera suspensions 16 at the first end 24 and to the body structure 12 with a fixation 28 at the second end 26. The longitudinal holding element 22 extends from the first 24 end to the second end 26 at least from the holding portion 18 towards the central point 14 with a length *L3.* The longitudinal holding element 22 is arranged for providing length compensation for a change of dimension of the body structure 12 due to thermal expansion. The thermal expansion is indicated with an arrow *T1* for the left longitudinal holding element 22 and with *T2* for the right longitudinal holding element 22. For better illustration, cameras 30 are indicated with hashed lines.

In a first option, the length compensation is provided by the longitudinal holding elements 22 extending across the central point 14 by the fixation 28 of the longitudinal holding element 22 attached to the body structure 12 beyond the central point 14 in relation to the respective camera suspension 16.

In a second option, the length compensation of the camera setup is provided by the longitudinal holding element 22 between the first end 24 and the second end 26 having a smaller thermal expansion than the body structure 12 between the central point 14 and the camera suspension 16.

In a variation, the first and the second option are combined.

Fig. 1 shows an option where the longitudinal holding elements 22 extend from the first 24 end to the second end 26 from the holding portion 18 towards the central point 14 with the length *L3* being less than the distance *L4.*

Fig. 2 shows another option where the longitudinal holding elements 22 extend from the first 24 end to the second end 26 from the holding portion 18 towards the central point 14 with the length *L3* being larger than the distance *L4.*

In a further option, not shown, the longitudinal holding elements 22 have a length *L3* equal to the distance *L4.*

As a result, a thermal stable design is provided, thus providing cameras at stable position. The solution can be referred to as passive solution where stabilization is achieved with thermal compensation in the suspension of the cameras.

The examples depicted in the figures show a symmetric setup in relation to the central point 14. It is noted that other examples are provided, in which the setup is non-symmetric in relation to the central point 14. For example, the examples depicted in the figures show a same length *L3* and a same distance *L4* for the at least two camera suspensions 16. In further examples, the length *L3* may vary, i.e. be different, while the distance *L4* is the same. In other examples, the length *L3* is the same while the distance *L4* may vary. In still further examples, the length *L3* varies and the distance *L4* also varies.

The term "mount 10" relates to a structure provided for the holding of a set of cameras. The mount 10 can also be referred to as mounting device or mounting arrangement.

The term "body structure 12" relates to a support structure for carrying load and ensuring a secure hold of the cameras 30. The body structure 12 can also be referred to as body or mounting structure or support structure.

The term "camera suspension 16" relates to a mount, e.g. a reception, for mounting a camera. The camera suspensions 16 can also be referred to as camera holding devices. The camera suspension 16 can also be referred to as camera holder or camera mount.

The term "fixation device 20" relates to an arrangement that securely fixes the camera suspensions 16 in relation to the body structure 12.

The term "longitudinal holding element 22" relates to an elongate structural part that is capable of holding the camera suspension 16 (with the mounted camera 30 when in use) in relation to the sliding movement of the camera suspension 16 on the body structure. The longitudinal holding elements 22 can also be referred to as holding bars or holding rods. The longitudinal holding elements 22 can also be referred to as thermal compensation bars or thermal compensation rods. The longitudinal holding element 22 is configured to extend freely in length direction between the first end 24 and the second end 26. In an example, the longitudinal holding element 22 is slidably or flexibly guided in length direction.

The term "fixation 28" relates to a secure connection of the longitudinal holding element 22 to the body structure 12.

The "central point 14" can also be referred to as neutral point, central reference point or thermal 0 (zero) center point.

In an example, the at least two camera suspensions 16 are arranged with the distance *L4* to the central point.

The proposed thermal compensated design (passive solution) avoids the use of active elements to compensate the thermal deviations, since additional active parts will increase the costs and an active compensation technology is needed with additional measuring technologies and compensation technologies. This is not needed with the proposed solution; the reliability will be better and the optimization of the design with respect look and feel will be better.

In an example, shown in Fig. 2, at least one of the longitudinal holding elements 22 extends across the central point 14 and the fixation 28 of the longitudinal holding element 22 is attached to the body structure 12 beyond the central point 14 in relation to the respective camera suspension 16, i.e. "beyond" when viewed from the first end 24. The fixation 28 is thus provided such that the longitudinal holding element 22 spans across the central point 22.

In an example it is provided that, with increasing temperature, the longitudinal holding element 22 expands at its first 24 end in a first direction of its extension in relation to the second end 26. With the fixation 28, the second end 26 is fixedly mounted to a part of the body structure 12 that expands in a second direction away from the central point 14. The first and the second direction are opposite to each other such that an expanding of the body structure 12 in the second direction at least partly compensates an expanding of the longitudinal holding element 22 in the first direction.

In Fig. 2, the first direction of the thermal expansion of the longitudinal holding element 22 is indicated for the left sliding camera suspension 16 with a first arrow *T3;* the second direction of the thermal expansion of the body structure 12 is indicated for the fixedly mounted fixation 28 with a second arrow *T5.* A third arrow *T6* indicates the thermal expansion of the body structure 12. However, due to the sliding mount of the camera suspension 16, this third thermal expansion *T6* has no direct effect on the position of the camera 30.

As a result, a camera 30 being hold by a camera suspension 16 can maintain its position in relation to the central point 14, since the camera suspension 16 slides in relation to the expanding body structure 12, while the expansion of the longitudinal holding element is compensated (at least partly) by the part of the body structure being arranged on the other side of the central point and thus expanding in an opposite direction.

In an example, also illustrated in Fig. 2 as an option, it is provided that the longitudinal holding element 22 is arranged such that the fixation 28 is arranged with a first distance *L1* to the central point 14 and the camera suspension 16 is arranged with a second distance *L2* to the central point 14. A ratio of the first distance *L1* and the second distance *L2* is based on a ratio of a thermal expansion of the body structure 12 and a thermal expansion of the longitudinal holding element 22.

In an example, an expansion ratio/difference for Aluminum and Invar is approximately 12 times. For a thermal stable design ΔL1 = ΔL2 is required, which results in L1 = (L1+L2) / 12. L1 and L2 can be tuned to different material and construction for precise shifting of the fixation points of the bars/rods.

For other material combinations the ratio will differ and can be optimized, this can be required with respect to heat performance, costs, and manufacturing technologies. Each camera holder can shift/move in a linear direction with respect to the camera cross housing so it will have a stable position related to the thermal "0" position.

In an example, the longitudinal holding element 22 provides a more stable relative position of the camera suspension 16 in relation to the central point 14 than the holding portions 18 in relation to the body structure 12.

In another example, the longitudinal holding element 22 between the first end 24 and the second end 26 is having a smaller thermal expansion than the body structure 12 between the central point 14 and the camera suspensions 16.

In an option, two camera suspensions 16 are provided arranged on two ends of a beam-like structure 32 provided by the body structure 12, which two ends are having the central point 14 between the two ends.

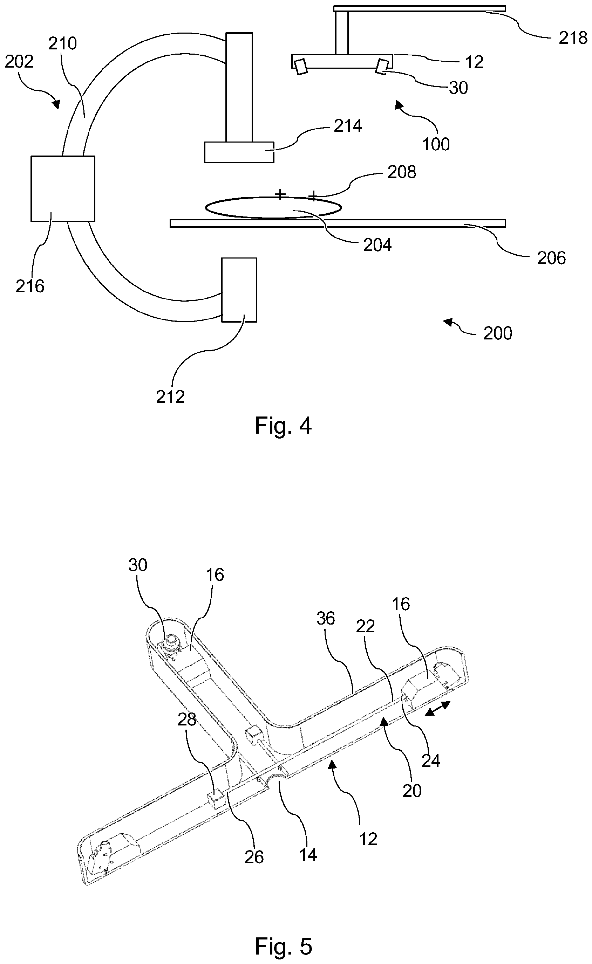

As an option, an elongated housing element (see Fig. 4 following) is provided.

In an example, each camera 30 is slidably arranged in an end portion of the elongated housing element and is fixed on a bar/rod positioned inside the housing element, the bar/rod being less thermally expandable than the housing element and being fixed to the housing element at a point a thermally optimized in the housing element.

In an example, the longitudinal holding element 22 is made from a material having a smaller thermal expansion than the body structure 12.

In an option, the longitudinal holding element is made from a nickel-iron alloy and the body structure is made from Aluminum. It is noted that material selection depends on production technologies, total weight and costs. In an example, the longitudinal holding element is made from Invar. Different materials can be selected for heat transfer, stability or manufacturing reasons.

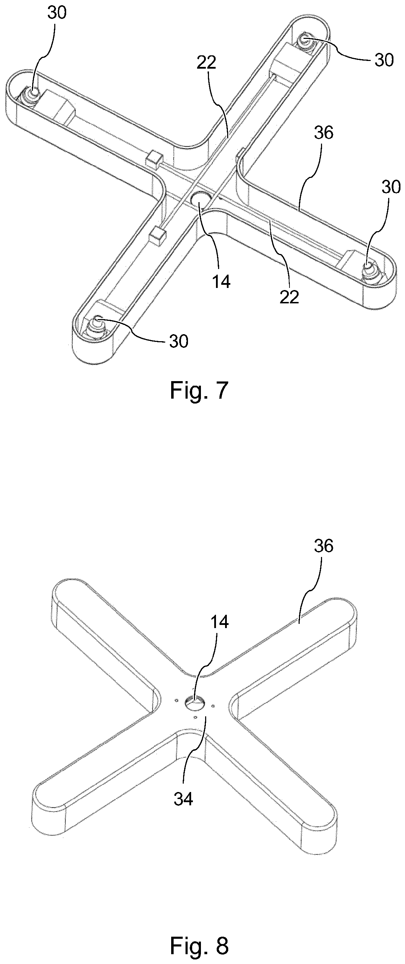

In an example (see also Fig. 8 and Fig. 9), a mounting device 34 is provided at the central point 14 for attaching the mount 10 to a support arrangement.

In a further example, also indicated in Fig. 1 and Fig. 2 as an option, a camera arrangement 100 is provided that comprises an example of the mount 10 according to one of the preceding examples and a plurality of the cameras 30. Each of the camera suspensions 16 is holding one of the plurality of cameras 30.

The camera arrangement 100 can be used as a part of a navigation technology used for mobile surgery, for example for navigation during spine or other orthopedic surgery based on a mobile C-arm X-ray imaging.

Also other applications are provided where these devices are also used, also in combination with a robot. Exemplary fields are urology, kidneys or lung structures.

The camera arrangement 100 can also be used for registration of surgical devices or robotics and combine this with patient registration. Thus, improved accuracies and better result in performance as well as improved workflows are provided.

In an example, cameras are embedded in a housing comprising a hollow elongated housing element made of a thermally expandable material.

In an example of the camera arrangement 100, a support is provided from which the body structure 12 of the mount is suspended. The support can also be referred to as support arrangement.

Fig. 3 shows steps of an example of a method 300 for holding cameras. The method 300 comprises the following steps:
In a first step 302, a mount for a camera arrangement of a medical navigation setup is provided. The mount comprises a body structure provided with a central point, which is configured for mounting the body structure to a support. The mount also comprises at least two camera suspensions slidably mounted to the body structure at holding portions, which holding portions are displaced in relation to the central point. The mount further comprises a fixation device for each of the at least two camera suspensions. Each of the fixation devices comprises a longitudinal holding element, which is attached to the respective camera suspension at a first end and which is attached to the body structure with a fixation at a second end. The longitudinal holding element extends from the first end to the second end at least from the holding portion towards the central point.

In a second step 304, the body structure is thermally expanding due to increasing thermal loads in a first direction.

In a third step 306, length compensation is provided by the longitudinal holding element for a change of dimension of the body structure due to thermal expansion.

The second and third step take place more or less simultaneously.

Fig. 4 shows an example of a medical imaging system 200. The medical imaging system 200 comprises a medical imaging arrangement 202, configured to acquire non-visible image data from a region of interest of a subject 204. In Fig. 4, the subject 204 is shown arranged on a subject support 206, for example an operation or examination table. Further, an example of the camera arrangement 100 according to the examples above is provided, configured to acquire visible image data from the region of interest of the subject 204. The system 200 further comprises a set of markers 208 that are visible by the medical imaging arrangement 202 and by the camera arrangement 100. The imaging system 200 is configured to provide a registration of visible images with image data of the medical imaging device based on the markers 208.

In an example, the medical imaging arrangement 202 is configured to acquire ultrasound image data of the subject.

In another example, the medical imaging arrangement is configured to acquire X-ray image data of the subject. Fig. 4 indicates a C-arm 210 with an X-ray source 212 and an X-ray detector 214 mounted to opposite ends of the C-arm 210. The C-arm 210 is movably supported by a C-arm mount 216.

The camera arrangement 100 is mounted to a suspending support 218.

In an example, the set of markers 208 comprises at least one of the group of skin markers for subject registration, device markers for tracking operating parameters of surgical devices like needle or drilling position tracking and robot markers for tracking and calibrating of robotic operation for accurate positioning and automated movements.

Fig. 5 shows a perspective view of a part of a camera arrangement 100 in a view from below. A housing 36 is provided that acts as the body structure 12. The body structure comprises two beams 38 that are provided in a crossing manner providing four beam ends having the central point 14 between the ends. Four camera suspensions 16 are provided, one arranged on each of the four ends of the beams.

In an example, four cameras 30 are arranged on support beams positioned with an angle of 90 degrees with respect to each other at a defined distance between the two camera opposite positions.

In an example (not shown in detail), four, six, eight or ten cameras 30 are arranged on support beams positioned with an angle of 30, 45 or 60 degrees with respect to each other at a defined distance between the two camera opposite positions.

In an example (not shown in detail), three camera suspensions 16 are provided. In a further example, five or more camera suspensions 16 are provided.

Optionally (also not shown in detail), the mount 10 comprises a housing with a second hollow elongated housing element, non-parallel to the first hollow elongated housing element, and positioned one to the other such that they have the same central portion (i.e. across configuration), the second hollow elongated housing element having similar arrangement, with two other cameras and a non-expandable support, as the first hollow elongated housing element.

In another example, the housing element is hollow, but it can also be flat with additional covers around it. This design aspect is also related to manufacturing aspects.

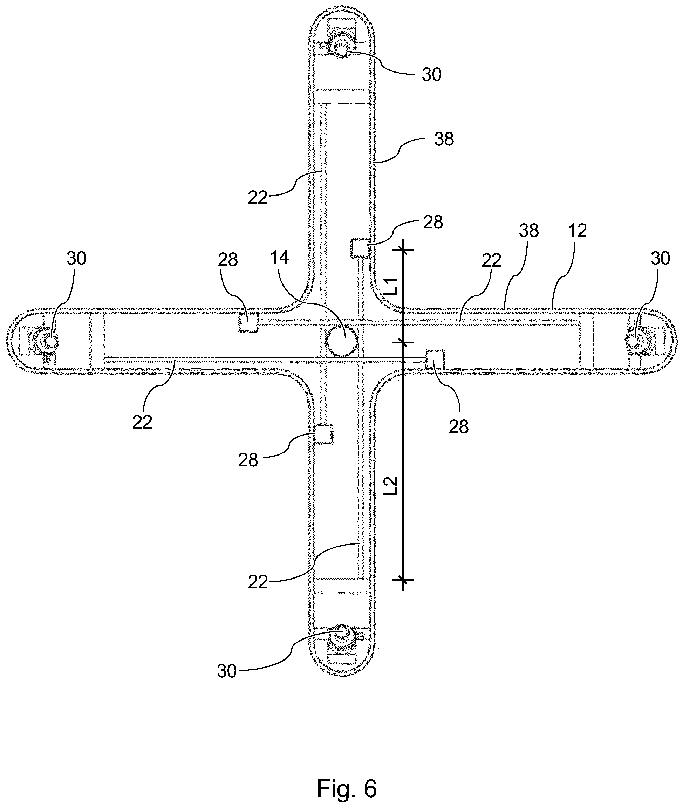

Fig. 6 shows a bottom view of the camera arrangement 100 of Fig. 5. As can be seen, the longitudinal holding elements 22 span across the central point 14.

Fig. 7 shows a further perspective view from below of the camera arrangement 100 with the four cameras 30 movably attached at the respective ends, but securely hold in the moving, i.e. sliding direction by the longitudinal holding elements 22.

Fig. 8 shows a perspective view from above of the camera arrangement 100. The mounting device 34 is indicated with four screw holes around the central point 14 in the center of the housing 36.

Fig. 9 shows a perspective view from above of the camera arrangement 100 mounted to a movable support arm 40. The support arm 40 provides the option for arranging the camera arrangement 100 in a desired position. Once arranged, the support arm 40 provides sufficient stability for securely holding the camera arrangement 100 in place, for example for observing an operation field.

The term "subject" may also be referred to as individual. The "subject" may further also be referred to as patient, although it is noted that this term does not indicate whether any illness or disease is actually present with the subject.

One cause for thermal expansion may be heat generated by the operation of the cameras. This does not only affect the internal optical setup of the camera, but also affects the position of the cameras to each other and may thus require a calibration procedure.

Another cause for thermal expansion may be thermal changes in the operation room.

The passive compensation assembly described above does not require a compensation each time the system is used. The correction is provided in an inherent manner.

In an example, X-ray images are provided to generate a volume for a 3D dataset. Based on the markers that are visible in X-ray an accurate correlation of the X-ray image data with visible image data from the cameras can be provided.

In another example, a 3D data set is available. Two 2D X-ray images are provided in an angle to each other. An overlay of the current X-ray images with the 3D dataset is possible based on the markers.

The examples depicted in the figures show a symmetric setup of the arrangement of the cameras in relation to the central point 14, for example with two opposing arms (in the beam-like version) of two pairs of opposing arms (in the cross-like version).

It is noted that other examples are provided, in which the setup provides three or five or more arms with cameras attached to the ends.

It is noted further that other examples are provided, in which the setup is non-symmetric in relation to the central point 14. For example, the arms with different lengths are provided.

In a still further example, a plate-like structure instead of beams is provided on which the cameras are mounted.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A mount (10) for a camera arrangement of a medical navigation setup, the mount comprising:
- a body structure (12) provided with a central point (14), which is configured for mounting the body structure to a support;
- at least two camera suspensions (16) slidably mounted to the body structure at holding portions (18), which holding portions are displaced in relation to the central point; and
- a fixation device (20) for each of the at least two camera suspensions;
wherein each of the fixation devices comprises a longitudinal holding element (22), which is attached to the respective camera suspensions at a first end (24) and which is attached to the body structure with a fixation (28) at a second end (26);
wherein the longitudinal holding element extends from the first end to the second end at least from the holding portion towards the central point; and
wherein the longitudinal holding element is arranged for providing length compensation for a change of dimension of the body structure due to thermal expansion.

2. Mount according to claim 1, wherein at least one of the longitudinal holding elements extends across the central point; and
wherein the fixation of the longitudinal holding element is attached to the body structure beyond the central point in relation to the respective camera suspensions.

3. Mount according to claim 1 or 2, wherein, with increasing temperature, the longitudinal holding element expands at its first end in a first direction of its extension in relation to the second end;
wherein, with the fixation, the second end is fixedly mounted to a part of the body structure that expands in a second direction away from the central point; and wherein the first and the second direction are opposite to each other such that an expanding of the body structure in the second direction at least partly compensates an expanding of the longitudinal holding element in the first direction.

4. Mount according to claim 1, 2 or 3, wherein the longitudinal holding element is arranged such that the fixation is arranged with a first distance (*L1*) to the central point and the camera suspension is arranged with a second distance (*L2*) to the central point; and
wherein a ratio of the first distance and the second distance is based on a ratio of a thermal expansion of the body structure and a thermal expansion of the longitudinal holding element.

5. Mount according to one of the preceding claims, wherein the longitudinal holding element provides a more stable relative position of the camera suspension in relation to the central point than the holding portions in relation to the body structure central point.

6. Mount according to one of the preceding claims, wherein the longitudinal holding element between the first end and the second end is having a smaller thermal expansion than the body structure between the central point and the camera suspensions.

7. Mount according to one of the preceding claims, wherein the longitudinal holding element is made from a material having a smaller thermal expansion than the body structure; and
wherein the longitudinal holding element is made from a nickel-iron alloy and the body structure is made from Aluminum.

8. Mount according to one of the preceding claims, wherein a mounting device (34) is provided at the central point for attaching the mounting arrangement to a support arrangement.

9. Mount according to one of the preceding claims, wherein two camera suspensions are provided arranged on two ends of a beam-like structure (32) provided by the body structure, which two ends are having the central point between the two ends.

10. Mount according to one of the preceding claims, wherein the body structure comprises two beams (38) that are provided in a crossing manner providing four beam ends having the central point between the two ends; and
wherein four camera suspensions are provided, one arranged on each of the four ends.

11. A camera arrangement (100) comprising:
- a mount (10) according to one of the preceding claims; and
- a plurality of cameras (30);
wherein each of the camera suspensions is holding one of the plurality of cameras.

12. Camera arrangement according to claim 11, wherein a support is provided to which the body structure of the mount is suspended.

13. A medical imaging system (200), comprising:
- a medical imaging arrangement (202), configured to acquire non-visible image data from a region of interest of a subject (204);
- a camera arrangement (100) according to claim 11 or 12, configured to acquire visible image data from the region of interest of the subject; and
- a set of markers (208) that are visible by the medical imaging arrangement and by the camera arrangement;
wherein the imaging system is configured to provide a registration of visible images with image data of the medical imaging device based on the markers.

14. Medical imaging system according to claim 13, wherein the set of markers comprises at least one of the group of:
- skin markers for subject registration;
- device markers for tracking operating parameters of surgical devices like needle or drilling position tracking; and
- robot markers for tracking and calibrating of robotic operation for accurate positioning and automated movements.

15. A method (300) for holding cameras, comprising the following steps:
- providing (302) a mount for a camera arrangement of a medical navigation setup, the mount comprising: a body structure provided with a central point, which is configured for mounting the body structure to a support; at least two camera suspensions slidably mounted to the body structure at holding portions, which holding portions are displaced in relation to the central point; and a fixation device for each of the at least two camera suspensions; wherein each of the fixation devices comprises a longitudinal holding element, which is attached to the respective camera suspension at a first end and which is attached to the body structure with a fixation at a second end; wherein the longitudinal holding element extends from the first end to the second end at least from the holding portion towards the central point;
- thermally expanding (304) the body structure due to increasing thermal loads in a first direction; and
- providing (306) length compensation by the longitudinal holding element for a change of dimension of the body structure due to thermal expansion.
